# EUROPÄISCHE PATENTSCHRIFT

(11) **EP 0 975 380 B1**
(45) Veröffentlichungstag und Bekanntmachung des Hinweises auf die Patenterteilung: **07.11.2001**
(21) Anmeldenummer: 98964344.0
(22) Anmeldetag: 17.04.1998
(51) Int. Cl.: A61L 31/00, A61K 6/00

(54) **MEMBRANSYSTEM ZUR GESTEUERTEN GEWEBEREGENERATION BEI ERKRANKUNGEN DES ZAHNHALTEAPPARATES**
MEMBRANE SYSTEM FOR CONTROLLED TISSUE REGENERATION IN CASES OF DISEASES OF THE PERIDONTIUM
SYSTEME DE MEMBRANE POUR LA REGENERATION CONTROLEE DE TISSUS DANS LE CAS D'AFFECTIONS DU PARODONTE

(30) Priorität: 22.04.1997 DE 19716815; 04.11.1997 DE 19748688
(43) Veröffentlichungstag der Anmeldung: 02.02.2000
(73) Patentinhaber: Gräber, Hans Georg, 52074 Aachen (DE); Lampert, Friedrich, 52074 Aachen (DE)
(72) Erfinder: Gräber, Hans Georg, 52074 Aachen (DE); Lampert, Friedrich, 52074 Aachen (DE)
(74) Vertreter: Schüssler, Andrea, Dr.
(86) Internationale Anmeldenummer: DE9801089
(87) Internationale Veröffentlichungsnummer: WO9847480

(56) Entgegenhaltungen:
- EP-A- 0 793 965
- WO-A-90/07308
- WO-A-90/11730
- WO-A-96/15795
- DE-A- 19 540 658
- US-A- 4 603 695
- US-A- 4 961 707

## Beschreibung

Die vorliegende Erfindung betrifft ein Membransystem zur Parodontitis-Therapie.

Die Entzündung der Gingiva, hervorgerufen durch mikrobielle Plaque, führt auch aufgrund von unkontrolliertem Wachstum des Saumepithels nach apikal und Bildung von Zahnfleischtaschen unbehandelt zu einem schrittweisen Verlust von Parodontalgewebe mit Zahnausfall. Davon sind der Alveolarknochen, das Wurzelzement und der desmodontale Faserapparat betroffen. Die bisher einzige regenerative Parodontitis-Therapie besteht in einem operativen Eingriff zur Entfernung der Zahnfleischtaschen, nachfolgender gründlicher Reinigung der betroffenen Stellen und Anlegen einer Membran aus expandiertem Polytetrafluorethylen (e-PTFE, Gore-Tex) um den Zahnhals. Anschließend wird das Zahnfleisch in seine ursprüngliche Lage zurückgebracht und das Implantat bedeckt. In einem zweiten operativen Eingriff wird nach ca. 4-6 Wochen das Implantat wieder entnommen. Die Nachteile dieser Methode liegen in einer möglichen bakteriellen Besiedlung der Membran mit Wundheilungsstörungen sowie in dem langwierigen und schmerzhaften Vorgehen mit 2 operativen Eingriffen.

WO-A-96/15795 betrifft Zusammensetzungen, die Lazaroide enthalten und deren Verwendung zur Verhinderung von Adhäsion.

US-A-4,961,707 betrifft zahnmedizinische Implantationsartikel, um die Regeneration des Periodontiums zu verbessern.

Die Aufgabe der vorliegenden Erfindung besteht deshalb in der Bereitstellung eines Mittels, das die obigen Nachteile bei der Parodontitis-Behandlung vermeidet.

Diese Aufgabe wird durch ein Membransystem gemäß Patentanspruch 1 gelöst. Vorteilhafte Ausgestaltungen ergeben sich aus den Unteransprüchen.

Erfindungsgemäß weist das erfindungsgemäße Membransystem eine resorbierbare Polymermembran sowie Anti-Adhäsionsmoleküle auf.

Als resorbierbare Polymermembran eignet sich jegliches aus Zahn- und Augemeinmedizin bekannte durch den Körper abbaubare, d.h. resorbierbare, physiologisch unbedenkliche Polymer. Insbesondere geeignet sind Poly(D,L-Lactid), Poly(D,L-lactid-co-trimethylencarbonat),Poly(ethylen-co-propylen),Poly(ethylen-co-vinylacetat) und Poly(D,L-lactid-co-glycolid) sowie deren Blends oder Mischungen. Diese sind käuflich erhältlich (z.B. von Fa. Boehringer Ingelheim). Die Polymere können bevorzugt durch übliche Verfahren oberflächenmodifiziert werden, um die Zelladhäsion zu verbessern. Dazu geeignet ist beispielsweise die plasmainduzierte Pfropfcopolymerisation mit 2-Hydroxyethylmethacrylat oder aber die Pfropfcopolymerisation mit Acrylsäure und anschließende kovalente Anbindung von Fibronectin.

Als Anti-Adhäsionsmoleküle eignen sich alle Moleküle, die die Wirkung von Adhäsionsmolekülen aufheben. Unter Adhäsionsmolekülen werden allgemein Moleküle verstanden, die eine wesentliche Rolle in der Zell-zu-Zell- und Zell-Matrix-Kommunikation spielen, insbesondere werden darunter die Integrine verstanden, die auch im Periodontium nachgewiesen wurden (Steffensen et al., J. Periodontol. 1992, 63: 584-592). Zur Aufhebung der Wirkung der Adhäsionsmoleküle eignen sich dazu kompetitive Proteine bzw. Peptide (sog. Disintegrine oder disintegrinähnliche Proteine bzw. Peptide) sowie gegen die Adhäsionsmoleküle gerichtete Antikörper, welche käuflich erhältlich sind. Beispielsweise sind dies monoklonale Maus-Antikörper gegen die Integrin-Untereinheit α-5 (Klon IOP 49c; Immunotech, Marseille), Integrin-Untereinheit β-4- (Klon 3E1; Biomol, Hamburg) Integrin-Untereinheit α-6 (Klon GoH3; Dianova, Hamburg) und Integrin-Untereinheit β-1 (Klon P4c10; Biomol, Hamburg). Insbesondere geeignet sind gegen die Integrin-Untereinheiten α-6 und β-1 gerichtete monoklonale Antikörper, einzeln und als Gemisch.

Die Anti-Adhäsionsmoleküle werden durch den Fachmann bekannte Verfahren auf bzw. in die Polymermembran gebracht. Dies kann zur kovalenten Oberflächenbindung der Anti-Adhäsionsmoleküle beispielsweise durch die Carbodiimid-Methode geschehen. Eine andere Möglichkeit ist die Vermischung der Anti-Adhäsionsmoleküle mit den Polymerkomponenten bei der Polymerherstellung oder die nachträgliche Oberflächenmodifizierung mittels plasmainduzierter Pfropfcopolymerisation. Zur gezielten Freigabe der Anti-Adhäsionsmoleküle werden die Polymermembransysteme lokal unterschiedlich damit bestückt.

Die Entzündung der Gingiva, hervorgerufen durch mikrobielle Plaque, führt auch aufgrund von unkontrolliertem Wachstum des Saumepithels nach apikal zu einer Verlagerung des Epithelansatzes nach apikal, dadurch wird eine Regeneration des kollagenen Faserapparates verhindert und die Haltefunktion des Parodontiums ist somit eingeschränkt. Für das Anhaften der Zellen an der Basalmembran sind von den Adhäsionsmolekülen insbesondere die Integrine verantwortlich. Sie binden an die extrazelluläre Matrix und bewirken dadurch eine Modifizierung der intrazellulären Genexpression, eine Änderung der zellulären Proliferation und Differenzierung. In der epithelialen Wundheilung dienen besonders die Integrin-untereinheiten α-6 und β-1 der Verbindung der in das Wundgebiet migrierenden Keratozyten mit allen extrazellulären Matrixproteinen der Basalmembran. Die Erfindung beruht nun darauf, daß eine Wachstumshemmung des Epithels bei gleichzeitiger Wachstumsstimulation im Bindegewebe erfolgen soll. Dadurch wird eine Regeneration des funktionellen Zahnhalteapparates und eine beschleunigte Wundheilung gefördert. Das Membransystem wird um den Zahnhals oder die Zahnhälse gelegt, bei denen die Entwicklung einer Parodontitis droht oder bei denen bereits ein erster operativer Eingriff vorgenommen worden ist, um die sich gebildeten Zahnfleischtaschen zu entfernen. In letzterem Fall läßt sich mit dem erfindungsgemäßen Membransystem dann der oben beschriebene zweite operative Eingriff vermeiden. Zur Anwendung kann eine zusammenhängende Membran oder ein System aus mehreren Membranuntereinheiten kommen. Bevorzugt ist, daß sich die Anti-Adhäsionsmoleküle nur in der Membranregion befinden, wo sie mit dem Saumepithel in Nachbarschaft treten. Dabei soll eine Wirkstoffkonzentration in Abhängigkeit vom verwendeten Anti-Adhäsionsmolekül erreicht werden. Beispielsweise werden 1:40 - 1:400 Verdünnungen von Antikörpern gegen die Integrinuntereinheiten α-6 und β-1 verwendet.

In einer bevorzugten Ausführungsform enthält das erfindungsgemäße Membransystem weiter Wachstumsfaktoren und/oder Zytokine, um gezielt das Wachstum des Parodontium-Bindegewebes zur Ausbildung eines funktionsfähigen Zahnhalteapparates anzuregen. Als Wachstumsfaktoren eignen sich die dem Fachmann bekannten, wie TGF-β oder EGF. Als Zytokine sind beispielsweise die Kolonie-stimulierenden Faktoren (CSF), Interleukine und Interferon-γ zu nennen. Die Wachstumsfaktoren und/oder Zytokine werden in bzw. an die Polymermembran auf die gleiche Weise gebracht wie vorstehend für die Anti-Adhäsionsmoleküle beschrieben. Auch für diese ist es vorteilhaft sich nur dort auf dem Membransystem zu befinden, wo sie die erwähnten Teile des Parodontiums beeinflussen können.

In einer bevorzugten Ausführungsform enthält das erfindungsgemäße Membransystem weiter Antibiotika. Diese Antibiotika dienen dazu, eine Besiedlung der Membran und des Kiefers mit bakteriellen Keimen zu verhindern und den Wundheilungsprozeß ohne bakterielle Entzündung ablaufen zu lassen. Als Antibiotika eignen sich grundsätzlich alle in der Zahn- und Kieferheilkunde dem Fachmann bekannten einsetzbaren Antibiotika. Dies sind insbesondere Antibiotika, die das anaerobe Keimspektrum betreffen, wie z.B. Tetracykline, Metronidazol, Makrolid-Antibiotika, Chinolone, Lincomycine und Chloramphenicol, und in Kombination mit herkömmlichen Antibiotika, wie Penicillinen, eingesetzt werden können. Ganz besonders bevorzugt ist Metronidazol. Die Antibiotika werden ebenso wie die Wachstumsfaktoren bzw. Zytokine und die Anti-Adhäsionsmoleküle in bzw. an die Polymermembran gebracht. Allerdings ist es bevorzugt, daß sie sich überall in dem Membransystem befinden, um eine flächendeckende vollständige antibakterielle Wirkung zu erreichen. Dabei ist eine Wirkstoffkonzentration von etwa 500-2000 ppm, insbesondere etwa 1000 ppm, im Serumspiegel wirksam.

Die vorliegende Erfindung eignet sich somit bestens für die unter dem Begriff "guided tissue regeneration" (GTR) bekannte Methode periodontale Defekte zu beheben.

Die Erfindung wird weiter durch das nachfolgende Beispiel erläutert.

### BEISPIEL

Biopsien von marginaler Gingiva, bestehend aus oralem Epithel und subepithelialem Bindegewebe, werden in Fragmenten von 1-2 mm² bei 37°C in 24-Well-Mikrotiterplatten mit RPMI 1640-Medium ergänzt mit L-Glutamin, 10% FCS, 50 µg/ml Gentamycin, 0,31 µg/100 ml Insulin und 5 µg/ml Hydrokortison kultiviert. Die Fragmente wurden in Gruppen geteilt und folgendermaßen verfahren:
I) ohne Zusatz von Antikörpern
II) Zusatz von irrelevanten Kontrollantikörpern (mouse-anti-rat-Immunglobulin NK 212-005-102; Dianova, Hamburg, Konzentration 1,8 mg/ml)
III) Zusatz von Antikörpern gegen Integrin-Untereinheit β-1 (Klon GoH3; Dianova, Hamburg)
IV) Zusatz von Antikörpern gegen Integrin-Untereinheit α-6 (Klon P4-c10; Biomol, Hamburg)
V) Zusatz von Antikörpern gegen beide Integrin-Untereinheiten in Kombination

Die Antikörper wurden in Mengen von jeweils 1:400 in Kulturmedium verdünnt und zugesetzt.

Bei täglichem Mediumwechsel und nach einheitlicher Kulturdauer von 8 Tagen erfolgte an Kryostatschnitten der Fragmente die histologische und immunhistologische Aufarbeitung.

Beurteilt wurden 1. die Epithelbildung auf der Wundmatrix, 2. Expression epithelialer Integrine und 3. rezeptorblockierende Antikörperreaktionen.

Während bei den Kontrollgruppen I und II (ohne und mit irrelevanten Antikörpern) eine vollständige Epithelisierung zu beobachten war, konnte über die erfolgreiche Integrinblockade von α-6 und/oder β-1 die Epithel-Migration inhibiert werden, wobei die Kombination beider Antikörper (Gruppe V) die besten Ergebnisse geliefert hat.

## Patentansprüche

1. Membransystem zur gesteuerten Geweberegeneration des Zahnhalteapparates aufweisend eine resorbierbare Polymermembran, **gekennzeichnet durch** das Vorhandensein von Anti-Adhäsionsmolekülen zur Aufhebung der Wirkung von Molekülen, die der Zell-zu-Zell und/oder Zell-zu-Matrix-Kommunikation dienen.

2. Membransystem nach Anspruch 1, wobei das resorbierbare Polymer ausgewählt ist aus: Poly(D,L-Lactid), Poly(D,L-lactid-co-trimethylencarbonat), Poly(ethylen-co-propylen), Poly(ethylen-co-vinylacetat) und Poly(D,L-lactid-co-glycolid) sowie deren Blends oder Mischungen.

3. Membransystem nach Anspruch 1 oder 2, wobei die Anti-Adhäsionsmoleküle ausgewählt sind aus zu Adhäsionsmolekülen kompetitiven Proteinen bzw. Peptiden sowie gegen Adhäsionsmoleküle gerichteten Antikörpern.

4. Membransystem nach Anspruch 3, wobei die Antikörper gegen die Integrin-Untereinheiten α-6 und/oder β-1 gerichtet sind.

5. Membransystem nach einem der Ansprüche 1 bis 4, wobei das Membransystem weiter Wachstumsfaktoren und/oder Zytokine aufweist.

6. Membransystem nach Anspruch 5, wobei der Wachstumsfaktor TGF-β1 ist.

7. Membransystem nach einem der Ansprüche 1 bis 6, wobei das Membransystem weiter ein oder mehrere Antibiotika aufweist.

8. Membransystem nach Anspruch 7, wobei das Antibiotikum Metronidazol ist.

9. Verwendung einer resorbierbaren Polymermembran auf der Anti-Adhäsionsmolekülen zur Aufhebung der Wirkung von Molekülen vorhanden sind, die der Zell-zu-Zell und/oder Zell-zu-Matrix-Kommunikation dienen, zur Herstellung eines Membransystems nach einem der Ansprüche 1 bis 8 zur gesteuerten Geweberegeneration des Zahnhalteapparates.

## Claims

1. A membrane system for the controlled tissue regeneration of the peridontium, comprising a reabsorbable polymer membrane, **characterized by** the presence of anti-adhesion molecules for compensating the effect of molecules serving the cell-to-cell and/or cell-to-matrix communication.

2. The membrane system according to claim 1, wherein the reabsorbable polymer is selected from: poly(D,L-lactide), poly(D,L-lactide-co-trimethylene carbonate), poly(ethylene-co-propylene), poly(ethylene-co-vinyl acetate) and poly(D,L-lacitde-co-glycolide) and the blends or mixtures thereof.

3. The membrane system according to claim 1 or 2, wherein the anti-adhesion molecules are selected from proteins or peptides competitive with respect to adhesion molecules and antibodies directed against adhesion molecules.

4. The membrane system according to claim 3, wherein the antibodies are directed against the integrin subunits α-6 and/or β-1.

5. The membrane system according to any of claims 1 to 4, wherein the membrane system further comprises growth factors and/or cytokines.

6. The membrane system according to claim 5, wherein the growth factor is TGF-β1.

7. The membrane system according to any of claims 1 to 6, wherein the membrane system further comprises one or more antibiotics.

8. The membrane system according to claim 7, wherein the antibiotic is metronidazole.

9. Use of a reabsorbable polymer membrane on which anti-adhesion molecules are present for compensating the effect of molecules serving the cell-to-cell and/or cell-to-matrix communication, for the production of a membrane system according to any of claims 1 to 8 for the controlled tissue regeneration of the peridontium.

## Revendications

1. Système à membrane pour la régénération tissulaire maîtrisée du parodonte, présentant une membrane polymère résorbable, **caractérisé par** la présence de molécules anti-adhésion pour supprimer l'effet de molécules qui servent à la communication d'une cellule à l'autre et/ou entre une cellule et la matrice.

2. Système à membrane selon la revendication 1, dans lequel le polymère résorbable est choisi parmi : poly(D,L-lactide), poly(D,L-lactide-carbonate de triméthylène), poly(éthylène-propylène), poly(éthylène-acétate de vinyle) et poly(D,L-lactide-glycolide) ainsi que leurs combinaisons ou mélanges.

3. Système à membrane selon la revendication 1 ou 2, dans lequel les molécules anti-adhésion sont choisies parmi des protéines ou des peptides entrant en compétition avec des molécules d'adhésion ainsi que des anticorps dirigés contre des molécules d'adhésion.

4. Système à membrane selon la revendication 3, dans lequel les anticorps sont dirigés contre les sous-unités de l'intégrine α-6 et/ou β-1.

5. Système à membrane selon l'une des revendications 1 à 4, ledit système à membrane présentant, en outre, des facteurs de croissance et/ou des cytokines.

6. Système à membrane selon la revendication 5, dans lequel le facteur de croissance est le TGF-β1.

7. Système à membrane selon l'une des revendications 1 à 6, ledit système à membrane présentant, en outre, un ou plusieurs antibiotiques.

8. Système à membrane selon la revendication 7, dans lequel l'antibiotique est le métronidazole.

9. Utilisation d'une membrane polymère résorbable sur laquelle des molécules anti-adhésion pour supprimer l'effet de molécules sont présentes et servent à la communication d'une cellule à l'autre et/ou entre une cellule et la matrice, pour la fabrication d'un système à membrane selon l'une des revendications 1 à 8 pour la régénération tissulaire maîtrisée du parodonte.
